# EUROPÄISCHE PATENTANMELDUNG

(11) **EP 3 202 379 A1**
(43) Veröffentlichungstag der Anmeldung: **09.08.2017**
(21) Anmeldenummer: 17154353.1
(22) Anmeldetag: 02.02.2017
(51) Int. Cl.: A61F 5/14, A43B 7/16, A43B 17/14

(54) **ORTHOPÄDISCHE SCHUHEINLAGE**

(30) Priorität: 08.02.2016 AT 500802016
(71) Anmelder: Orthopädie Pilz GmbH, 4320 Perg (AT); Brunner, Peter, 4020 Linz (AT)
(72) Erfinder: Brunner, Peter, 4020 Linz (AT); Pilz, Stefan, 4320 Perg (AT)
(74) Vertreter: KLIMENT & HENHAPEL

(57) **Zusammenfassung**

Orthopädische Schuheinlage mit einem Fersenbereich, einem mittleren Bereich und einem Zehenbereich, die in Längsrichtung aufeinanderfolgen, bei der vorgeschlagen wird, dass im Fersenbereich eine pelottenförmige Erhöhung (1) angeordnet ist, die einen in Richtung der Außenkontur des Fersenbereiches abfallenden Fersenbereichsabschnitt (2) sowie einen in Richtung des mittleren Bereiches (6) abfallenden Abschnitt (3) bildet, wobei der mittlere Bereich (6) als ein gegenüber dem Zehenbereich (5) erhöhter Bereich ausgeführt ist, der über einen stufenförmigen Übergang (7) in den Zehenbereich (5) übergeht. Die erfindungsgemäße Schuheinlage behandelt die mit der Plantarfaszie zusammenhängenden Fußbeschwerden ursächlich und stellt daher eine verbesserte Therapie insbesondere für die *Fasczitis Plantaris* dar.

## Beschreibung

Die Erfindung betrifft eine orthopädische Schuheinlage mit einem Fersenbereich, einem mittleren Bereich und einem Zehenbereich, die in Längsrichtung aufeinanderfolgen, gemäß dem Oberbegriff von Anspruch 1.

Orthopädische Schuheinlagen werden zur Vorbeugung und Therapie von Fußbeschwerden verwendet, unter anderem von Beschwerden, die im Zusammenhang mit der Plantaraponeurose stehen, die auch als Plantarfaszie bezeichnet wird. Die Plantarfaszie ist eine Bindegewebsstruktur, die sich vom Fersenbein bis zu den Zehengrundgelenken erstreckt und so das Längsgewölbe des Fußes abspannt. Sie entspringt im Bereich der Ferse von der Unterseite des Fersenbeinhöckers. Von dort aus zieht sie sich zunächst in Form eines Mittelstreifens in Richtung des Vorderfußbereiches. Der Mittelstreifen teilt sich dabei in fünf Längszügel (*Fasciculi longitudinales*) auf, die V-förmig auseinanderlaufen. Im Bereich der Mittelfußköpfchen gibt es Querzüge (*Fasciculi transversi*), die diese Längszügel miteinander verbinden. Die fünf Längszügel enden unter anderem im Bereich der Gelenkkapseln der Zehengrundgelenke.

Fußbeschwerden können etwa aufgrund von Entzündungen der Plantarfaszie entstehen (*Fasczitis Plantaris*) oder aufgrund von Verklebungen oder Überbelastungen der Plantarfaszie. Andere Beschwerden sind etwa als Hohl- oder Spreizfüße bekannt, oder sind auf verkürzte Sehnen an der Fußsohle zurückzuführen. Zur Vorbeugung oder Behandlung dieser Beschwerden sind orthopädische Einlagen bekannt, die zumeist lediglich der Freilegung und Entlastung der Ferse dienen, aber nicht die Ursache der Schmerzen an der Ferse oder der Fußsohle beseitigen.

Es ist daher das Ziel der Erfindung eine orthopädische Schuheinlage bereitzustellen, die mit der Plantarfaszie zusammenhängende Fußbeschwerden ursächlich behandelt und daher eine verbesserte Therapie insbesondere für die *Fasczitis Plantaris* ermöglicht.

Dieses Ziel wird durch die Merkmale von Anspruch 1 erreicht. Anspruch 1 bezieht sich auf eine orthopädische Schuheinlage mit einem Fersenbereich, einem mittleren Bereich und einem Zehenbereich, die in Längsrichtung aufeinanderfolgen, bei der erfindungsgemäß vorgesehen ist, dass im Fersenbereich eine pelottenförmige Erhöhung angeordnet ist, die einen in Richtung der Außenkontur des Fersenbereiches abfallenden Fersenbereichsabschnitt sowie einen in Richtung des mittleren Bereiches abfallenden Abschnitt bildet, wobei der mittlere Bereich als ein gegenüber dem Zehenbereich erhöhter Bereich ausgeführt ist, der über einen stufenförmigen Übergang in den Zehenbereich übergeht.

Der durch die perlottenförmige Erhöhung in Richtung der Außenkontur des Fersenbereiches abfallende Fersenbereichsabschnitt nimmt bei Anwendung der Einlage das Fersenbein des Anwenders auf und übt dabei einen Druck auf den Ansatz der Plantarfaszie in Faserverlaufsrichtung aus. Der in Richtung des mittleren Bereiches abfallende Abschnitt der perlottenförmigen Erhöhung bietet Raum für Ausdehnung und entlastet dadurch die Plantarfaszie. Aufgrund des stufenförmigen Überganges, der durch eine individuelle Anpassung der erfindungsgemäßen Einlage auf den jeweiligen Anwender so geformt ist, dass er hinter den Mittelfußköpfchen platziert ist, wird einerseits Druck auf die Plantarfaszie ausgeübt, insbesondere auf deren Querzüge (*Fasciculi transversi*), und andererseits eine Zugwirkung, die insbesondere den Mittelstreifen und deren fünf Längszügel (*Fasciculi longitudinales*) der Plantarfaszie dehnt. Diese Dehnung wird dabei durch eine Fixierung des Fersenbeins bewerkstelligt, die durch den in Richtung der Außenkontur des Fersenbereiches abfallenden Fersenbereichsabschnitt erreicht wird.

Vorzugsweise bildet der in Richtung der Außenkontur des Fersenbereiches abfallende Fersenbereichsabschnitt hierfür eine dem Fersenbein zugeordnete Aufnahmemulde, die dem Fersenbein des jeweiligen Anwenders individuell angepasst wird. Der stufenförmige Übergang verläuft dabei vorzugsweise in Querrichtung in einer der Lage der Mittelfußköpfchen zugeordneten Form. Die Lage der Mittelfußköpfchen variiert nämlich entlang der Breite des Fußes, sodass der Verlauf des stufenförmigen Überganges in Querrichtung der Einlage vorzugsweise ebenfalls der Lage der Mittelfußköpfchen des jeweiligen Anwenders individuell nachgebildet wird.

Die erfindungsgemäße Einlage wirkt somit statisch und dynamisch durch Ausübung einer Kombination von Zug- und Druckkräften auf die Plantarfaszie. Sie wird dabei durch Verwendung entsprechend formstabiler Materialien gefertigt, vorzugsweise aus einem EVA-Material mit einer Härte von 45-60 Shore.

Die Erfindung wird in weiterer Folge anhand eines Ausführungsbeispiels mithilfe der beiliegenden Figuren näher erläutert. Es zeigen hierbei die
Fig. 1 eine perspektivische Ansicht einer erfindungsgemäßen Schuheinlage, und die
Fig. 2 eine Seitenansicht der erfindungsgemäßen Schuheinlage mit einer schematisch dargestellten Anatomie des Fußes.

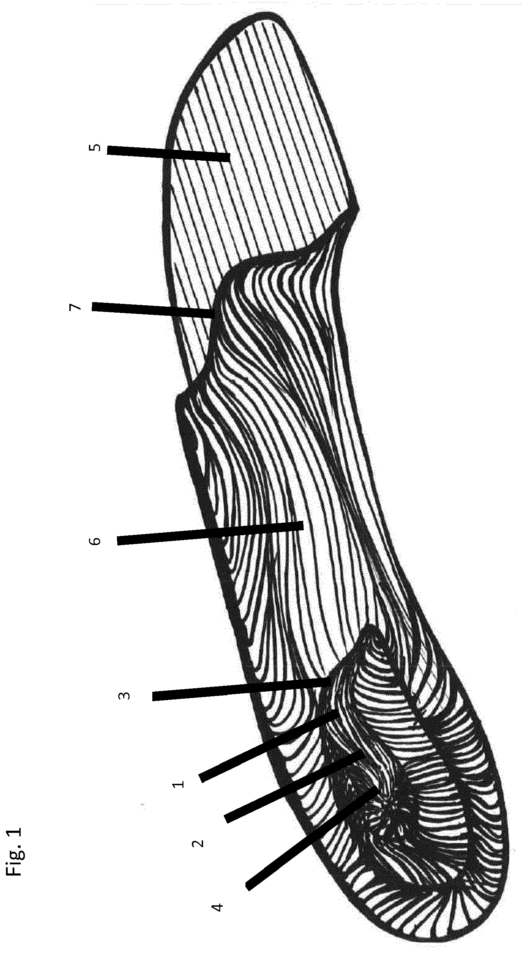

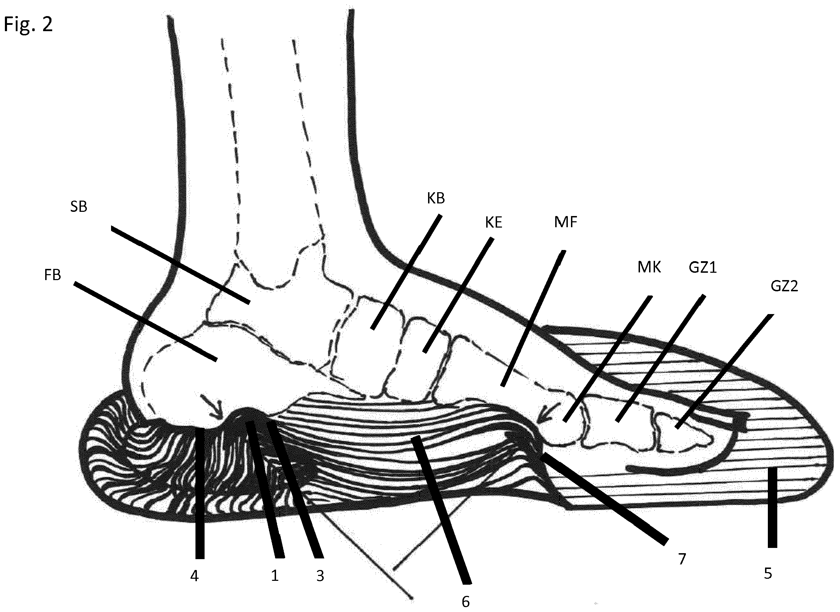

In den Fig. 1 ist zunächst die pelottenförmige Erhöhung 1 im Fersenbereich der Einlage ersichtlich. Eine Pelotte ist in der Regel ein ballenförmiges, meist halbkugeliges Element, das zur Kompression von Gewebe genutzt wird. In der Orthopädietechnik werden diese Elemente zur Formung von Erhöhungen in Einlagen verwendet. Bei Verwendung von EVA (Ethylenvinylacetat)-Materialien werden Einlagen auch oft mithilfe einer Oberflächenformung mithilfe von Fräsen hergestellt, wobei ebenfalls pelottenförmige Erhöhungen 1 gefertigt werden können. Die pelottenförmige Erhöhung 1 bildet einen in Richtung der Außenkontur des Fersenbereiches abfallenden Fersenbereichsabschnitt 2 sowie einen in Richtung des mittleren Bereiches 6 abfallenden Abschnitt 3, wobei der in Richtung der Außenkontur des Fersenbereiches abfallende Fersenbereichsabschnitt 2 eine dem Fersenbein zugeordnete Aufnahmemulde 4 bildet. Der mittlere Bereich 6 der Einlage ist als ein gegenüber dem Zehenbereich 5 erhöhter Bereich ausgeführt, der über einen stufenförmigen Übergang 7 in den Zehenbereich 5 übergeht. Die Aufnahmemulde 4 wird in ihrer Lage und Ausdehnung vorzugsweise individuell angepasst. Ebenso wird der Verlauf des stufenförmigen Überganges 7 in Querrichtung der Einlage der Lage der Mittelfußköpfchen MK (siehe Fig. 2) des jeweiligen Anwenders individuell nachgebildet.

Die Wirkung einer solchen Einlage wird anhand der Fig. 2 erläutert, in der auch schematisch die Anatomie des Fußes eingezeichnet ist. Der Fuß lässt sich in drei Abschnitte unterteilen, nämlich den Abschnitt der Fußwurzel (*Tarsus*), den Abschnitt des Mittelfußes (*Metatarsus*) und den Abschnitt der Großzehe (*Halux*) mit den vier anderen Zehen (*Digiti pedis*).

Die Fußwurzel umfasst sieben Fußwurzelknochen (*Ossa tarsi*), nämlich das Fersenbein FB (*Calcaneus*) mit dem Fersenbeinhöcker, der auch der Achillessehne als Ansatz dient, das Sprungbein SB (*Talus*), das Kahnbein KB (*Os naviculare*), das innere, mittlere und äußere Keilbein (*Ossa cuneiformia*), von denen in der Seitenansicht der Fig. 2 nur das innere Keilbein KE ersichtlich ist, und das Würfelbein (*Os cuboideum*), das in der Seitenansicht der Fig. 2 ebenfalls nicht ersichtlich ist.

Der Mittelfuß besteht aus den fünf Mittelfußknochen (*Ossa metatarsalia*), von denen in der Seitenansicht der Fig. 2 nur der innerste Mittelfußknochen MF mit seinem Mittelfußköpfchen MK ersichtlich ist.

Die Großzehe wird aus zwei Knochen GZ1, GZ2 gebildet, und die vier anderen Zehen aus jeweils drei Knochen (in der Fig. 2 nicht ersichtlich).

Der Fuß ist durch eine Vielzahl kräftiger Bänder, Sehnen und Muskeln so fest verspannt, dass sich ein Längs- und ein Quergewölbe ergeben, wobei in der Seitenansicht der Fig. 2 nur das Längsgewölbe ersichtlich ist. Das Längsgewölbe bildet einen ausgeprägten Bogen in Längsrichtung des Fußes, so dass der Spann deutlich hervortritt. Das Quergewölbe spannt sich quer über Anteile der Mittelfußknochen MF und der Fußwurzel.

Bei Anwendung einer Einlage gemäß der Fig. 1 nimmt der durch die perlottenförmige Erhöhung 1 in Richtung der Außenkontur des Fersenbereiches abfallende Fersenbereichsabschnitt 2 das Fersenbein FB des Anwenders auf und übt dabei einen Druck auf den Ansatz der Plantarfaszie in Faserverlaufsrichtung aus. Der in Richtung des mittleren Bereiches 6 abfallende Abschnitt 3 der perlottenförmigen Erhöhung 1 bietet Raum für Ausdehnung und entlastet dadurch die Plantarfaszie. Aufgrund des stufenförmigen Überganges 7, der durch eine individuelle Anpassung der erfindungsgemäßen Einlage auf den jeweiligen Anwender so geformt ist, dass er hinter den Mittelfußköpfchen MK platziert ist, wird einerseits Druck entlang den in der Fig. 2 eingezeichneten Pfeilrichtungen auf die Plantarfaszie ausgeübt, und andererseits eine Zugwirkung, die die Plantarfaszie entlang eines Bogens dehnt, der in der Fig. 2 mithilfe der mit Pfeilen gekennzeichneten Kreissehnen angedeutet wird. Diese Dehnung wird dabei durch eine Fixierung des Fersenbeins FB bewerkstelligt, die durch den in Richtung der Außenkontur des Fersenbereiches abfallenden Fersenbereichsabschnitt 2 erreicht wird.

Die erfindungsgemäßen Einlagen werden etwa aus EVA-Materialien mit einer Shore A-Härte von 45 oder 60 gefräst. Die Produktion erfolgt etwa mithilfe einer 3-Achsen-Fräsmaschine oder eines 5-Achsen-Roboterarms anhand eines von einem Orthopädietechniker angefertigten Fußabdruckes des zukünftigen Anwenders. Mithilfe des Fräsvorganges wird unter exakter Reproduktion des Fußabdruckes die pelottenförmige Erhöhung 1 und der stufenförmige Übergang 7 gefertigt.

Die erfindungsgemäße Schuheinlage behandelt die mit der Plantarfaszie zusammenhängenden Fußbeschwerden ursächlich und stellt daher eine verbesserte Therapie insbesondere für die *Fasczitis Plantaris* dar.

## Patentansprüche

1. Orthopädische Schuheinlage mit einem Fersenbereich, einem mittleren Bereich und einem Zehenbereich, die in Längsrichtung aufeinanderfolgen, **dadurch gekennzeichnet, dass** im Fersenbereich eine pelottenförmige Erhöhung (1) angeordnet ist, die einen in Richtung der Außenkontur des Fersenbereiches abfallenden Fersenbereichsabschnitt (2) sowie einen in Richtung des mittleren Bereiches (6) abfallenden Abschnitt (3) bildet, wobei der mittlere Bereich (6) als ein gegenüber dem Zehenbereich (5) erhöhter Bereich ausgeführt ist, der über einen stufenförmigen Übergang (7) in den Zehenbereich (5) übergeht.

2. Orthopädische Schuheinlage nach Anspruch 1, **dadurch gekennzeichnet, dass** der in Richtung der Außenkontur des Fersenbereiches abfallende Fersenbereichsabschnitt (2) eine dem Fersenbein (FB) zugeordnete Aufnahmemulde (4) bildet.

3. Orthopädische Schuheinlage nach Anspruch 1 oder 2, **dadurch gekennzeichnet, dass** der stufenförmige Übergang (7) in Querrichtung in einer der Lage der Mittelfußköpfchen (MK) zugeordneten Form verläuft.

4. Orthopädische Schuheinlage nach einem der Ansprüche 1 bis 3, **dadurch gekennzeichnet, dass** sie aus einem EVA-Material mit einer Härte von 45-60 Shore gefertigt ist.
